Europäisches Patentamt

European Patent Office

Office européen des brevets

(19)

(11) Veröffentlichungsnummer: **0 331 772**
**A1**

(12) **EUROPÄISCHE PATENTANMELDUNG**

(21) Anmeldenummer: 88103624.8

(22) Anmeldetag: 08.03.88

(51) Int. Cl.⁴: **G01F 1/36 , A61B 5/08 ,
G01F 1/38**

(43) Veröffentlichungstag der Anmeldung:
13.09.89 Patentblatt 89/37

(84) Benannte Vertragsstaaten:
AT CH DE ES FR GB IT LI NL SE

(71) Anmelder: PPG HELLIGE B.V.
I.B.C.-Weg 1
NL-5683 PK Best(NL)

(72) Erfinder: de Beer, R.H.M.
Domtorenstraat 18
NL-5037 AS Tilburg(NL)
Erfinder: Kuypers, M.H.
Schoolstraat 53
NL-5561 AH Riethoven(NL)
Erfinder: Holland, J.
v.d. Duyn v. Maasdamstraat 4
NL-4191 GC Geldermalsen(NL)
Erfinder: Schotte, H.
Goudrenetstraat 11
NL-5363 NT Eindhoven(NL)

(74) Vertreter: Patentanwälte TER MEER - MÜLLER
- STEINMEISTER
Mauerkircherstrasse 45
D-8000 München 80(DE)

(54) Differenzdruckmesser für bidirektionale Gasströme.

(57) Der Differenzdruckmesser für bidirektionale Gasströme, insbesondere für die Messung der Atemströmung und des Atmungsdrucks, besteht aus der Kombination eines röhrenförmigen passiven mechanischen Wandlerelements (1) mit unmittelbar angesetztem elektronischem Differenzdruckmesser (25). Das passive Wandlerelement (1) enthält zwischen zwei Meßanschlüssen (52a, 52b) ein elastisches Blendenelement (60), das durch einen einseitig angelenkten polygonalen Ausschnitt einer quer zur Gasströmung gespannten Membran (61) gebildet ist und durch elastisch rückstellende Auslenkung einen strömungsgeschwindigkeitsproportionalen dynamischen Strömungswiderstand bildet. Der Differenzdrucksensor (25) ist mit einer elektronisch geregelten Heizeinrichtung (6) ausgestattet, so daß trotz vorzugsweise vorgesehener Filter (2A, 2B) eindringende Feuchtigkeit ohne Einfluß auf die IC-Drucksensorelemente (7, 8) bleibt. Der Differenzdruckmesser zeichnet sich durch ein sehr lineares, frequenzgangfreies Meßsignal über einen großen Luftdurchsatzbereich von annähernd 0 bis 200 l/min, rasches Ansprechverhalten und leichte Auswechselbarkeit aus.

FIG.3

Xerox Copy Centre

## Differenzdruckmesser für bidirektionale Gasströme

Die Erfindung bezieht sich auf Gasströmungsmesser und betrifft insbesondere einen Differenzdruckmesser für bidirektionale Gasströme nach dem Oberbegriff des Patentanspruchs 1, der insbesondere als Gerät für die Messung der Atemströmung und des Atmungsdrucks sowie bei der Beatmungsüberwachung eingesetzt wird.

Ein Gasströmungsmesser mit einem Differenzdruckmesser nach dem Oberbegriff des Patentanspruchs 1 ist in der US-A-4 083 245 beschrieben. Dieser bekannte Differenzdruckmesser weist ein in einen Gasströmungskanal eingesetztes und als "passiver Wandler" zu bezeichnendes Strömungswiderstandselement auf, das im wesentlichen aus einem zwischen zwei Meßanschlüssen angeordneten elastischen Blendenelement besteht, das durch einen einseitig angelenkten polygonalen Ausschnitt einer quer zur Gasströmung gespannten Membran gebildet ist und einen elastisch rückstellenden dynamischen Strömungswiderstand bildet, der sich durch unterschiedlich starke Auslenkung des Blendenelements in Strömungsrichtung weitgehend strömungsgeschwindigkeitslinear ändert. Der polygonale Schnitt des Blendenelements kann beispielsweise die Form eines Rhombus aufweisen, der entlang einer Schmalseite mit dem übrigen Teil der Membran verbunden ist; es wird insoweit insbesondere auf Fig. 2 der genannten US-A-4 083 245 verwiesen. Gegenüber den aus US-A-2 989 866 und US-A-3 989 037 bekannten elastisch verformbaren Strömungswiderständen solcher passiver Wandler besitzt ein nach der US-A-4 083 245 aufgebauter Gasströmungsmesser den Vorteil, daß das erzielbare Druckdifferenzsignal in Abhängigkeit von der Gasströmungsgeschwindigkeit bzw. dem Gasdurchsatz linearer ist.

Der Erfindung liegt die Aufgabe zugrunde, einen Differenzdruckmesser der beschriebenen Art in verschiedener Hinsicht zu verbessern.

So hat sich, wie sich aus dem Diagramm der beigefügten Fig. 1(B) ersehen läßt, gezeigt, daß sich mit dem bekannten Strömungsmesser, insbesondere dem nach US-A-4 083 245, im Bereich kleinerer Luftdurchsätze bis etwa 80 l/min unter bestimmten konstruktiven Voraussetzungen, die allerdings der US-A-4 083 245 nicht entnehmbar sind, weil dort keine Meßanordnung angegeben ist, eine gut lineare Abhängigkeit des Druckverlusts bzw. Differenzdrucks erreichen läßt. Bei größeren Gas-(Luft-)Durchsätzen jedoch ist einerseits der Differenzdruck nicht mehr linear zur Strömungsgeschwindigkeit und zum anderen treten Instabilitäten auf, die zunächst nicht erklärbar waren.

Genauere Untersuchungen, die erst mit einer als Teil der vorliegend beschriebenen Erfindung verwirklichten Meßanordnung möglich wurden, bestätigten dann die Vermutung, daß das sogenannte Totvolumen der die Meßanschlüsse des passiven Wandlers mit dem elektrischen Differenzdruckmesser im vom Meßort entfernten Gerät verbindenden, nachgiebig verformbaren Schläuche, das Totvolumen in den Meßanschlüssen selbst und eventuell der Innenraum im Strömungsrohr des Differenzdruckmessers eine Ursache für die Fluktuationen bzw. den unerwünschten Frequenzgang des Meßsignals sein könnten. Bei dem bekannten Strömungsmesser nach der US-A-4 083 245 ist der (dort nicht beschriebene) elektronische Meßwandler (Drucktransducer) im vom passiven Meßelement oder Wandler entfernten Ort in ein Gerät eingebaut und beide Teile, nämlich das passive Wandlerelement einerseits und der elektronische Meßwandler andererseits, sind über Kunststoffschläuche verbunden. Insbesondere die Flexibilität dieser Verbindungsschläuche, Kondenswasser in den Schläuchen und dergleichen, beeinträchtigen den Frequenzgang und außerdem ergibt sich aufgrund des Abstands zwischen dem in ein Strömungsrohr eingebauten passiven Wandler und dem elektronischen Meßwandler im entfernten Gerät eine Verzögerungszeit des Meßwerts.

Aus dieser Erkenntnis folgte die der Erfindung zugrunde liegende Idee, nämlich bei einem Differenzdruckmesser für bidirektionale Gasströme, insbesondere für die Messung der Atemströmung und des Atmungsdrucks sowie zur Beatmungsüberwachung einen unmittelbar an die Meßanschlüsse des passiven Wandlers anzusetzenden elektronischen Differenzdrucksensor vorzusehen, der ein zur Druckdifferenz zwischen den Meßanschlüssen proportionales elektrisches Signal liefert.

Bereits durch die Vermeidung von Totvolumina im Differenzdruckmeßkreis und durch Vermeidung von flexibel verformbaren Anschlußschläuchen, wurde eine wesentliche Verbesserung des Frequenzgangs des Differenzdrucksignals erreicht.

Eine weitere Verbesserung wurde mit der Beseitigung von Feuchtigkeitseinflüssen im Gehäuse des oder der Drucksensorelement erreicht, nämlich dadurch, daß eine temperaturgeregelte beheizbare und vorzugsweise elastisch im Sensorgehäuse aufgehängte Kammer für die als IC-Bausteine ausgeführten Drucksensorelemente verwendet wurde.

Ergänzend zu dieser Maßnahme wurden hydrophob wirkende Folienfilter unmittelbar in die sehr kurzen Verbindungswege zwischen dem passiven Wandler und dem unmittelbar angesetzten Differenzdrucksensor eingebaut, die als Bakteriensperre wirken.

Mit der Erfindung wurden gegenüber bekannten Gasströmungsmessern für den hier vorwiegend vorgesehenen Anwendungszweck folgende Verbesserungen erreicht:

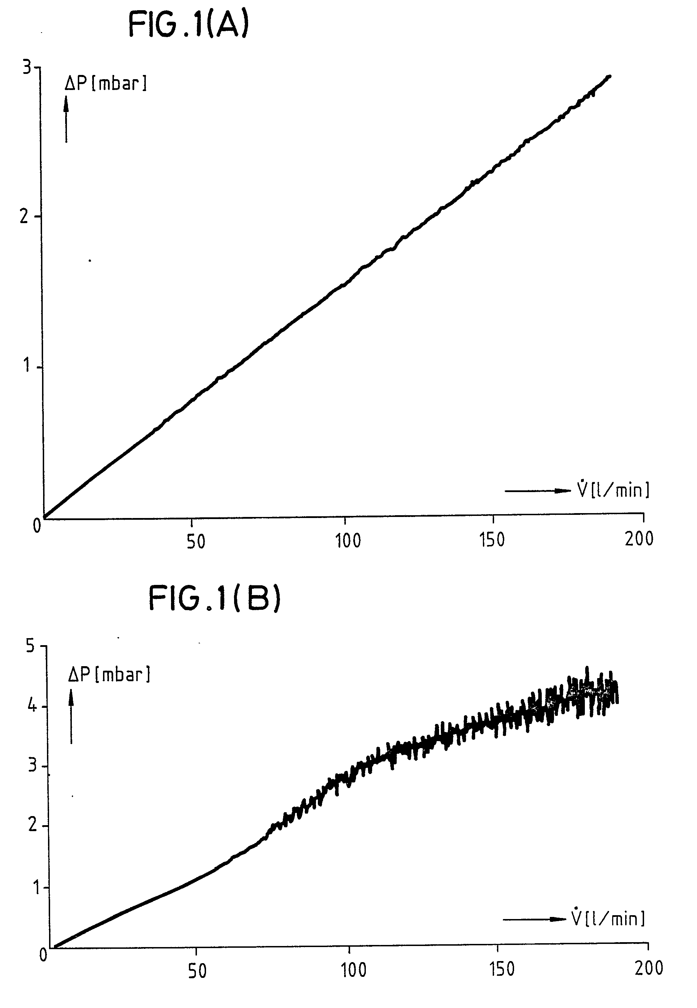

- Der maximale Druckverlust (Differenzdruck) ist über einen großen Luftdurchsatz oder Strömungsgeschwindigkeitsbereich von annähernd 0 bis 200 l/min kleiner als einige mbar und beträgt, wie sich beispielsweise aus Fig. 1(A) ersehen läßt, bei strenger Linearität des Meßsignals, nur etwa 3 mbar.

- Feuchtigkeitseinflüsse auf das Meßergebnis sind durch geregelte Übertemperatur im Differenzdrucksensor weitgehend beseitigt.

- Durch den zusätzlichen Einbau von hydrophoben frequenzgangneutralen Filtern, die gleichzeitig undurchdringlich sind für Bakterien, wurde außerdem eine Rückwirkung vom schwierig zu sterilisierenden Differenzdrucksensor auf den Patientenkreis ausgeschlossen.

Die Erfindung und vorteilhafte Einzelheiten werden nachfolgend unter Bezug auf die Zeichnungen in einer beispielsweisen Ausführungsform näher erläutert. Es zeigen:

Fig. 1 zwei vergleichende Diagramme für das Druckdifferenzsignal in Abhängigkeit vom Gasdurchsatz beim besten derzeit bekannten Stand der Technik (B) bzw. bei der Erfindung (A);

Fig. 2 das Prinzipblockschaltbild einer Differenzdruckmeßanordnung;

Fig. 3 den prinzipiellen Aufbau eines passiven Wandlerelements mit gemäß der Erfindung unmittelbar angesetztem Differenzdrucksensor;

Fig. 4 die schematische Querschnittdarstellung der Kombination aus passivem Wandler mit erfindungsgemäß unmittelbar angesetztem Differenzdruckmesser;

Fig. 5 die Teilschnittdarstellung des Differenzdrucksensors gemäß Fig. 3;

Fig. 6 die teilweise aufgebrochene Darstellung der Draufsicht auf den Sensor nach Fig. 5; und

Fig. 7 die vergrößerte Schnittdarstellung eines in eine Innenkammer in einem Gehäuse eingebauten Differenzdrucksensors.

Die Vergleichsdiagramme (A) und (B) der Fig. 1 wurden bereits oben erläutert.

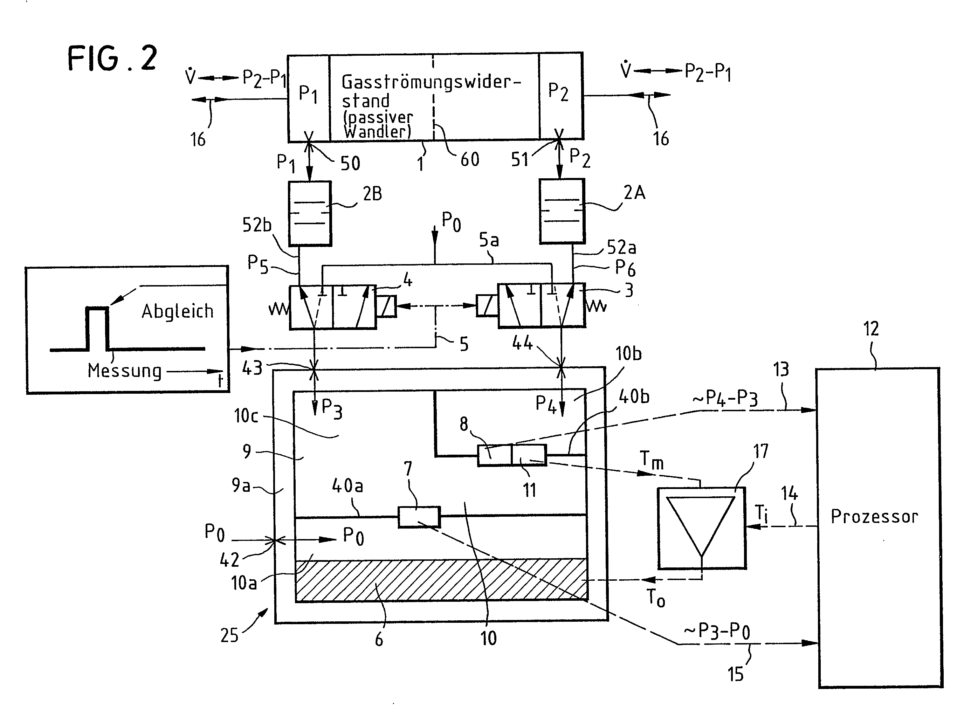

Im Prinzipblockschaltbild der Fig. 2 kennzeichnet Bezugshinweis 1 einen Gasströmungswiderstand oder passiven Wandler, der, von einigen wesentlichen Detailabwandlungen abgesehen, die Gegenstand einer datumsgleich eingereichten europäischen Patentanmeldung sind, hinsichtlich der grundsätzlichen konstruktiven Gestaltung im wesentlichen dem in US-A-4 083 245 beschriebenen Strömungswiderstand zur Erzielung eines linearisierten Differenzdrucks entspricht. Die schematisch angedeuteten Meßanschlüsse 50 bzw. 51 liegen in jeweiliger Strömungsrichtung vor bzw. hinter einem in Fig. 2 nur gestrichelt angedeuteten Blendenelement 60, das der Gasströmung 16 einen definierten strömungsgeschwindigkeitsabhängigen Widerstand entgegensetzt. Die Meßanschlüsse 50, 51 sind mit unmittelbar angesetzten oder eingebauten hydrophob wirkenden, für Bakterien undurchdringlichen Antikontaminationsfiltern 2A bzw. 2B, beispielsweise aus Teflonfolie, versehen. Nach den Filtern 2A, 2B folgen elektrisch oder mechanisch steuerbare Ventile 3 bzw. 4, über die in einen Innenraum 9 im Inneren eines allgemein mit Bezugshinweis 25 angegebenen Differenzdrucksensors zu Anschlüssen 43 bzw. 44 wahlweise die Druckwerte $P_5$ bzw. $P_6$ oder in der gestrichelt eingezeichneten Ventilstellung über eine Leitung 5a ein Bezugsdruck $P_0$ (z. B. Atmosphärendruck) durchschaltbar ist. Die Druckwerte $P_5$ bzw. $P_6$ entsprechen in der eingezeichneten Position den Druckwerten $P_1$ bzw. $P_2$ an den Meßanschlüssen 50 bzw. 51 unmittelbar vor bzw. hinter dem Gasströmungswiderstand 60, jedoch gegebenenfalls vermindert um den frequenzgangneutralen Druckdifferenzwert, den die Filter 2A bzw. 2B verursachen. In dieser für die Messung gültigen Position der Ventile 3 und 4 gilt auch für die angegebenen Drücke $P_3$ in einer Kammer 10c bzw. $P_4$ in einer Kammer 10b: $P_3 = P_5$ und $P_4 = P_6$. In der gestrichelt eingezeichneten Abgleichposition gilt dann: $P_3 = P_0$ bzw. $P_4 = P_0$. Der Innenraum 9 ist durch eine in Fig. 2 schematisch angedeutete erste Trennwand 40a und eine zweite Trennwand 40b in drei Kammern 10a, 10b und 10c unterteilt. Die in Fig. 2 untere Kammer 10a ist mit dem Bezugsdruck $P_0$ beaufschlagt. Im Bereich einer Durchbrechung der ersten Trennwand 40a zwischen den Kammern 10a und 10c ist ein erster Drucksensor 7 angeordnet, während eine weitere Durchbrechung der zweiten Trennwand 40b durch einen zweiten Drucksensor 8 überdeckt ist, der außerdem mit einem integrierten Temperatursensor 11 versehen ist, der die Temperatur im Innenraum 9 mißt. Der Innenraum 9 kann über eine in Fig. 2 ebenfalls nur schematisch angedeutete Heizeinrichtung 6 beheizt werden, wobei die von einem Temperaturregelverstärker 17 abgegebene Heizleistung durch den Temperaturwert $T_0$ (indirekt) angedeutet ist. Der Heizregelverstärker 17 wird eingangsseitig durch den vom integrierten Temperatursensor 11 festgestellten Temperaturwert $T_m$ beaufschlagt. Ein Temperaturvorgabewert $T_i$ wird über eine Leitung 14 von einem die Gesamtanordnung kontrollierenden Prozessor 12 vorgegeben, beispielsweise als voreinstellbarer Temperaturwert. Der erste Drucksensor 7 mißt die Druckdifferenz zwischen dem Druck $P_3$ am Einlaß 43 und dem Bezugsdruck $P_0$ am Bezugsdruckeingang 42. Der zweite Drucksensor 8 mißt den Differenzdruck zwischen dem Druckwert $P_4$ am Einlaß 44 und dem Druckwert $P_3$ am Einlaß 43. Die Drucksensoren 7, 8 sind über Leitungen 13, 15 auf den Prozessor 12 geschaltet. Über den Temperaturregelverstärker 17 und die Heizeinrichtung 6 wird die Temperatur der als Chips ausgebildeten Drucksensoren 7, 8 auf einer Temperatur gehalten, die über der Gastemperatur im Bereich des Gasströmungswiderstands 60 liegt. Das die Temperatur $T_0$ bestimmende Heizstromsignal vom Verstärker 17 wird so geregelt, daß die Differenz zwischen der (vorgebbaren) Betriebstemperatur $T_i$ und der Temperatur $T_m$ am Chip des Drucksensors 8 konstant bleibt ($T_i$ -$T_m$ = constant).

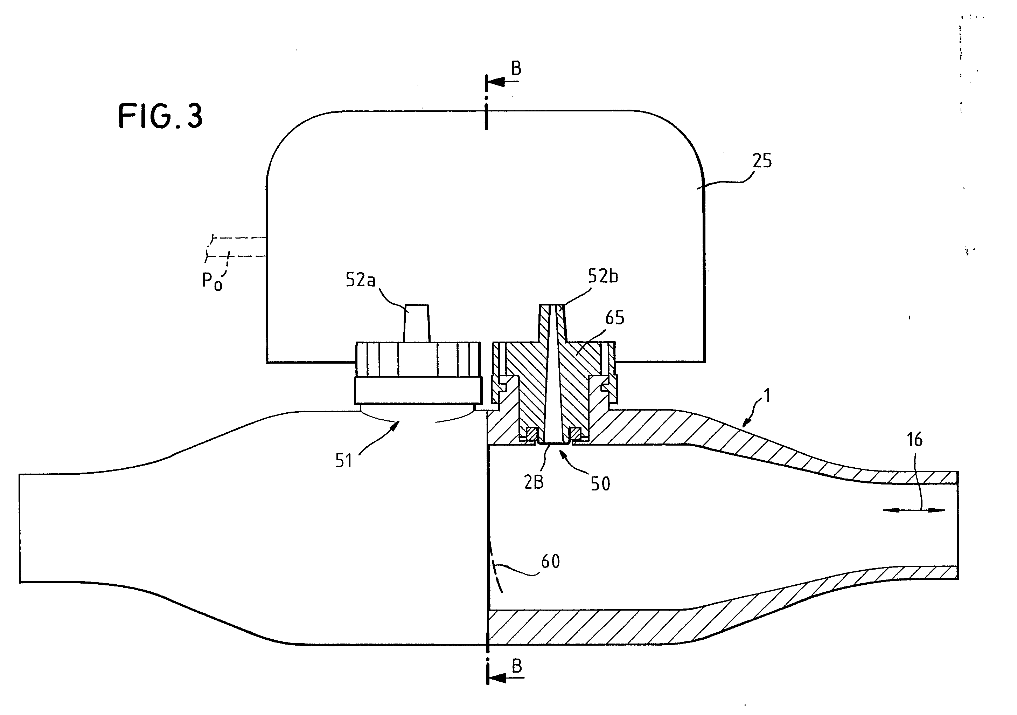

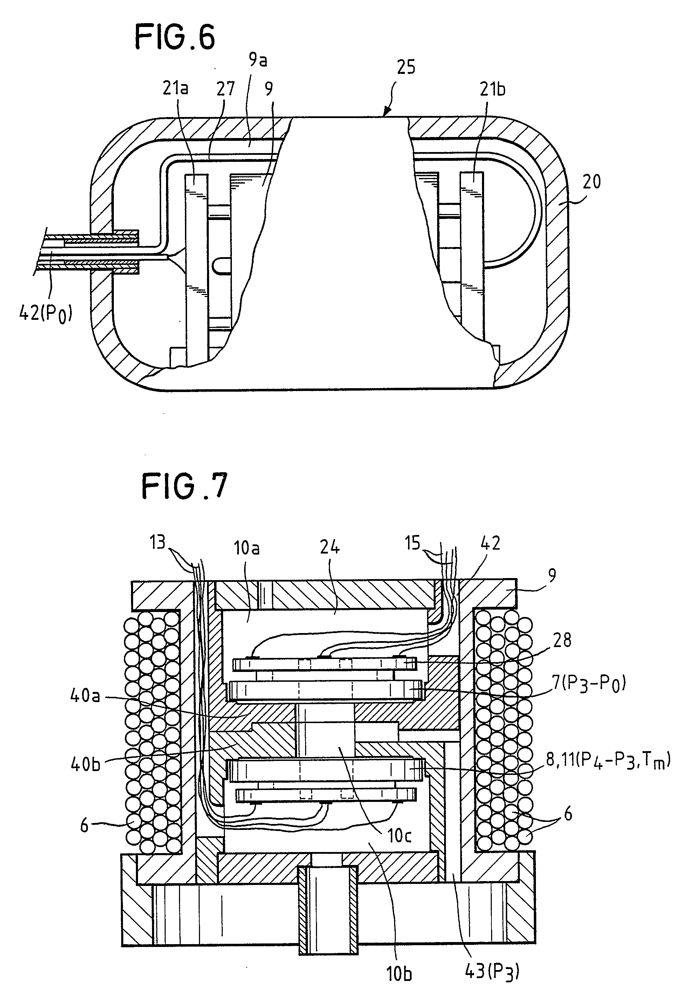

Die Fig. 3 veranschaulicht den Grundgedanken der Erfindung, nämlich das passive Wandlerelement 1 mit unmittelbar an die Meßanschlüsse 50 und 51 angesetztem elektronischem Differenzdrucksensor 25. Der Differenzdrucksensor 25 ist mit seinen Eingängen 43, 44 (siehe Fig. 2 und 7) unmittelbar an die Meßanschlüsse 52a bzw. 52b des passiven Wandlers 1 angeschlossen unter Zwischenschaltung der beispielsweise in den passiven Wandler 1 oder das Gehäuse des Differenzdrucksensors 25 eingesetzten (Membran-)Filter 2A, 2B aus hydrophober Folie. Die Verbindung zwischen dem Differenzdruck sensor 25 und dem passiven Wandler 1 erfolgt über einen Konus 65; der Anschluß zwischen dem Verschluß der austauschbaren Filter 2A, 2B und dem passiven Wandler 1 ist durch einen Luer-Lock-Verschluß verwirklicht. Es kommen auch andere Anschlußmöglichkeiten in Frage. Der besondere Vorteil der hier erwähnten und erprobten Anschlußverbindung des Druckdifferenzsensors 25 ist darin zu sehen, daß die vorzugsweise als Einwegteile eingesetzten Filterelemente 2A, 2B auf einfache Weise ausgetauscht werden können.

Der besondere Vorteil des unmittelbaren Anschlusses des Differenzdrucksensors 25 an den passiven Wandler 1 besteht darin, daß Totvolumina in flexiblen Anschlußschläuchen und Verbindungsstücken weitgehend vermieden werden, so daß ein nachteiliger Einfluß auf den Frequenzgang des Drucksensorsignals, insbesondere bei höheren Luftdurchsätzen, vermieden wird.

Es kann je nach konstruktiver Gestaltung unter praktischen Gesichtspunkten zweckmäßig sein, die Filter 2A, 2B in den gegebenenfalls einstückig auszuführenden passiven Wandler zu integrieren und als auswechselbare Baueinheit auszuführen. Gedacht ist dabei auch an die Herstellbarkeit als Einwegteil.

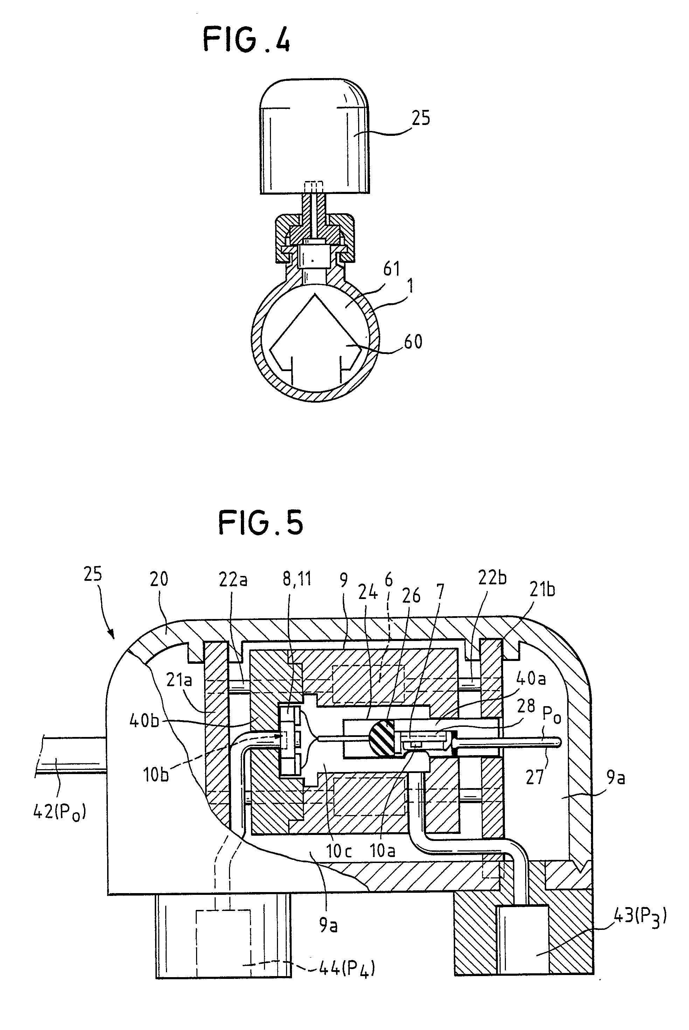

Die schematische Teilschnittdarstellung der Fig. 4 zeigt -anders als die vergrößerte Wiedergabe der Fig. 3 - in etwa natürlicher Größe eine Schnittdarstellung gesehen in Richtung der Pfeile B-B in Fig. 3. Worauf es hier ankommt, ist die Darstellung des in der Ebene der spannungsfrei eingespannten Membran 61 liegenden Blendenelements 60 in der Mitte der axialen Erstreckung zwischen den beiden Hälften des rohrartigen Gehäuses des passiven Wandlers 1. Der Schnitt der Blende 60 läßt eine fünfeckige Konfiguration erkennen. Durch zwei in Vertikalrichtung stehende Einschnitte wird einerseits eine hohe Flexibilität des Blen denelements 60 erreicht und außerdem läßt sich über die Einschnittiefe eine im Hinblick auf die Linearität des Druckdifferenzsignals günstige Federkonstante einstellen. Die besondere Gestaltung der Membran und des Blendenelements ist Gegenstand der bereits erwähnten datumsgleich eingereichten europäischen Patentanmeldung der gleichen Anmelderin.

Die Fig. 5 und 6 lassen den Innenaufbau des Differenzdrucksensors 25 erkennen. Das äußere Gehäuse 20 des Sensors 25 besteht aus einem wärmeisolierenden Material. Der die Drucksensoren 7 und 8 aufnehmende Innenraum 9 ist an zwei Vertikalwänden 21a und 21b über Heizdrähte 22a und 22b aufgehängt, die einerseits als Stromzuführung für das elektrische Heizelement 6 und andererseits zur elastischen Aufhängung des Drucksensorgehäuses 9 unter Berücksichtigung eines Freiraums 9a dienen. Die Elemente der Heizeinrichtung 6 können, wie in Fig. 7 veranschaulicht, aus einer Widerstandsheizwicklung oder beispielsweise auch aus Zenerdioden oder diskreten Widerständen bestehen. Der zweite Drucksensor 8 mit integriertem Temperatursensor 11 ist als Chip unter Zwischenschaltung eines Siliciumsubstrats auf eine Wand des Sensorgehäuses 9 aufgesetzt. Mindestens diese Wand besteht aus einem Material, das einerseits gut wärmeleitend ist und andererseits einen Temperaturausdehnungskoeffizienten aufweist, der demjenigen des für die Chips der Drucksensoren 8 bzw. 7 verwendeten Siliciummaterials entspricht, beispielsweise das unter dem Handelsnamen "Vacodil 36" bekannte Material.

Der erste Drucksensor 7 befindet sich in einem mittels einer Dichtung 26 luftdicht abgetrennten Gehäuseteil 24, das über eine Leitungsverbindung 27 unterseitig, d. h. über die Kammer 10a, mit dem am Einlaß 42 herrschenden Druck $P_0$ beaufschlagt wird. Der Chipträger für den Drucksensor 7 ist mit Bezugshinweis 28 gekennzeichnet.

Die Fig. 7 verdeutlicht in genauerer Detaildarstellung eine abgewandelte Ausführungsform der Anordnung der Drucksensoren 7 und 8 in dem von der Wicklung der Heizeinrichtung 6 umgebenen Drucksensorgehäuse 9. Ersichtlicherweise wird der Drucksensor 8 mit integriertem Temperatursensor 11 einerseits (von unten) mit dem Druck $P_4$ und andererseits (von oben) mit dem Druck $P_3$ beaufschlagt, der auch die Unterseite des Drucksensors 7 beaufschlagt. Letzterer Drucksensor 7 wird ober-

seitig durch den Druck $P_0$ in der Kammer 10a beaufschlagt. Die Versorgungs- und Signalabführleitungen für die Drucksensoren 7 und 8 bzw. den Temperatursensor 11 sind mit 13 bzw. 15 angegeben.

## Ansprüche

1. Differenzdruckmesser für bidirektionale Gasströme, insbesondere für die Messung der Atemströmung und des -drucks, mit einem in einen Gasströmungskanal eingesetzten passiven Wandler (1), der ein zwischen zwei Meßanschlüssen (50, 51) eingebautes elastisches Blendenelement (60) aufweist, das einen strömungsgeschwindigkeitsabhängigen (dynamischen) Strömungswiderstand bildet, **gekennzeichnet durch** einen unmittelbar an die Meßanschlüsse (50, 51) des passiven Wandlers (1) angesetzten elektronischen Differenzdrucksensor (25), der ein zur Druckdifferenz ($P_2 - P_1$ bzw. $P_5 - P_6$) zwischen den Meßanschlüssen (50, 51) proportionales elektrisches Signal liefert.

2. Differenzdruckmesser nach Anspruch 1, **dadurch gekennzeichnet,** daß zwischen Meßanschlüssen des passiven Wandlers (1) und dem Differenzdrucksensor (25) frequenzgangneutrale, hydrophob wirkende Filter (2A, 2B) eingesetzt sind.

3. Differenzdruckmesser nach Anspruch 2, **dadurch gekennzeichnet,** daß die Filter (2A, 2B) zusätzlich bakterizid wirksam sind.

4. Differenzdruckmesser nach Anspruch 1, **dadurch gekennzeichnet,** daß das elastische Blendenelement (60) durch einen einseitig angelenkten, polygonalen Ausschnitt einer quer zur Gasströmungsrichtung gespannten Membran gebildet ist.

5. Differenzdruckmesser nach Anspruch 1, **dadurch gekennzeichnet,** daß im Differenzdrucksensor (25) ein als druckempfindliches Siliciumhalbleiterbauelement verwirklichter Drucksensor (8) mit integriertem Temperatursensor (11) zur Steuerung eines Regelverstärkers (17) einer Temperaturregelung für den Innenraum (10) des den Drucksensor (8, 11) umgebenden Gehäuses (9) dient.

6. Differenzdruckmesser nach Anspruch 1, **dadurch gekennzeichnet,** daß der Differenzdrucksensor (25) zwei als Siliciumhalbleiterbauelemente ausgebildete Drucksensoren (7, 8) aufweist, von denen einer (8) zur Messung des Differenzdrucks ($P_2 - P_1$ bzw. $P_5 - P_6$) zwischen den Meßanschlüssen (50, 51) und der andere (7) zur Messung einer Vergleichsdruckdifferenz ($P_3 - P_0$) zwischen einem der Meßanschlüsse und einem Bezugsdruck ($P_0$) dient.

7. Differenzdruckmesser nach Anspruch 6, **dadurch gekennzeichnet,** daß
- der Innenraum (10) des Drucksensorgehäuses drei Kammern (10a, 10b, 10c) aufweist, die gegeneinander druckdicht abgeschlossen sind, von denen
-- die erste Kammer (10a) mit dem Bezugsdruck ($P_0$),
-- die zweite Kammer (10b) mit einem dem Druck ($P_2$) am einen Meßanschluß (51) gleichen oder proportionalen Druckwert ($P_4$) und
-- die dritte Kammer (10c) mit einem dem Druck ($P_1$) am anderen Meßanschluß (50) gleichen oder proportionalen Druckwert ($P_3$) beaufschlagbar ist,
- der eine Drucksensor (7) in die Trennwand (40a) zwischen der ersten und der dritten Kammer (10a, 10c) eingesetzt ist und den Differenzdruck zwischen dem anderen Maßanschluß (50) und dem Bezugsdruck ($P_0$) mißt, und daß
- der andere Drucksensor (8) in die Trennwand (40b) zwischen der zweiten und der dritten Kammer (10b, 10c) eingesetzt ist und den Differenzdruck zwischen den beiden Meßanschlüssen (50, 51) erfaßt.

8. Differenzdruckmesser nach Anspruch 7, **gekennzeichnet durch** eine elektrisch oder mechanisch steuerbare Ventilan ordnung (3, 4), durch welche die zweite bzw. die dritte Kammer (10b bzw. 10c) mit dem einen bzw. dem anderen Druckwert ($P_4$ bzw. $P_3$) oder beide genannte Kammern mit dem Bezugsdruck ($P_0$) beaufschlagbar sind.

9. Differenzdruckmesser nach Anspruch 6 oder 7, **dadurch gekennzeichnet,** daß wenigstens einem der Drucksensoren (8) ein Temperatursensor (11) zugeordnet ist, der zur Steuerung einer Temperaturregelung (17) für den Innenraum (10) dient.

10. Differenzdruckmesser nach Anspruch 5 oder 9, **dadurch gekennzeichnet,** daß im Drucksensorgehäuse eine Heizeinrichtung (6) angeordnet ist und daß die den Innenraum (10) umgrenzenden Kammerwände aus einem Material hoher thermischer Leitfähigkeit und/oder mit linearem Temperaturexpansionskoeffizienten bestehen, der auf den thermischen Expansionskoeffizienten des Siliciummaterials der Druck- bzw. Temperatursensorelemente (7, 8, 11) angepaßt ist.

11. Differenzdruckmesser nach Anspruch 10, **dadurch gekennzeichnet,** daß dieser elastisch und wärme- und/oder elektrisch isoliert in einem äußeren Gehäuse (20) aufgehängt ist.

## FIG.1(A)

## FIG.1(B)

FIG. 2

FIG.3

# FIG.4

# FIG.5

# FIG.6

# FIG.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | FR-A-2 290 656 (N.V. PHILIPS' GLOEILAMPENFABRIEKEN) * Insgesamt * | 1,4,6,7 | G 01 F 1/36 A 61 B 5/08 G 01 F 1/38 |
| A | --- | 5 | |
| D,Y | FR-A-2 360 063 (RESEARCH DEVELOPMENT CORP.) * Seite 3, Zeile 12 - Seite 4, Zeile 21; Figuren 1,2 * ----- | 1,4,6,7 | |

RECHERCHIERTE SACHGEBIETE (Int. Cl.4)

G 01 F
A 61 B

Der vorliegende Recherchenbericht wurde für alle Patentansprüche erstellt

| Recherchenort | Abschlußdatum der Recherche | Prüfer |
|---|---|---|
| DEN HAAG | 14-11-1988 | ROSE A.R.P. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
.........................................................................
& : Mitglied der gleichen Patentfamilie, übereinstimmendes
    Dokument

EPO FORM 1503 03.82 (P0403)